(19) Europäisches Patentamt
European Patent Office
Office européen des brevets

(11) **EP 3 978 596 A1**

(12) **EUROPEAN PATENT APPLICATION**

(43) Date of publication:
**06.04.2022 Bulletin 2022/14**

(21) Application number: **20306145.2**

(22) Date of filing: **02.10.2020**

(51) International Patent Classification (IPC):
*C12M 3/06* (2006.01)     *B01F 3/04* (2006.01)
*C12M 1/00* (2006.01)     *C12M 1/34* (2006.01)

(52) Cooperative Patent Classification (CPC):
**C12M 29/26; B01F 23/29; C12M 23/16; C12M 41/34; G05D 11/00**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
• **SORBONNE UNIVERSITE**
  **75006 Paris (FR)**
• **Centre national de la recherche scientifique**
  **75016 Paris (FR)**
• **Institut Curie**
  **75005 Paris (FR)**
• **Fluigent**
  **94270 Le Kremlin-Bicêtre (FR)**

(72) Inventors:
• **CESAR, William**
  **75009 Paris (FR)**
• **BOUQUEREL, Charlotte**
  **69003 Lyon (FR)**
• **VERHULSEL, Marine**
  **92240 Malakoff (FR)**
• **VIOVY, Jean-Louis**
  **75013 Paris (FR)**
• **DESCROIX, Stéphanie**
  **75014 Paris (FR)**

(74) Representative: **Bandpay & Greuter**
  **30, rue Notre-Dame des Victoires**
  **75002 Paris (FR)**

Remarks:
Amended claims in accordance with Rule 137(2) EPC.

(54) **DEVICE FOR PROVIDING A LIQUID MEDIUM WITH A CONTROLLED FLOW RATE AND WITH A CONTROLLED DISSOLVED GAS CONCENTRATION**

(57)     The present invention relates to a device (2) for providing a liquid medium with a controlled flow rate and with a controlled concentration of a dissolved gas, the device (2) comprising:
- a liquid reservoir (4) provided with:
- at least one gas inlet (7);
- at least one gas outlet (8); and
- at least one liquid outlet (9);

- an admission line (5) configured to provide a gaseous medium into the liquid reservoir (4), the admission line (5) being connected to the at least one gas inlet (7) and comprising at least one control valve (12); and
an emission line (6) configured to withdraw the gaseous medium from the liquid reservoir (4), the emission line (6) being connected to the at least one gas outlet (8) and comprising at least one control valve (15).
   The present invention further relates to an assembly comprising said device, and to a method for providing a liquid medium with a controlled flow rate and with a controlled concentration in a dissolved gas.

Fig. 1

**Description**

TECHNICAL FIELD

The present invention relates to a device for providing a liquid medium with a controlled flow rate and with a controlled concentration of a dissolved gas. The invention also relates to an assembly comprising said device and to a method implemented in said assembly.

TECHNICAL BACKGROUND

Cell cultures such as microfluidic cell cultures constitute an important technology for applications such as drug screening, tissue culturing, toxicity screening, and biologic research. This technology can provide improved biological function, higher-quality cell-based data, reduced reagent consumption, and lower cost.

In addition, as in vitro samples may closely represent their in vivo characteristics, handling, characterization, culturing, and visualization of cells or other biologically or chemically active materials (such as beads coated with various biological molecules) has become increasingly valued in the fields of drug discovery, disease diagnoses and analysis, and a variety of other therapeutic and experimental work.

Therefore, it has become essential to be able to control the environment of the cell culture in order to mimic at best the in vivo conditions. Among such conditions, the concentration of the culture in oxygen represents an important factor that should be controlled and measured. Various systems are proposed nowadays to control the oxygen concentration in a cell culture. However, such systems present drawbacks such as gaseous control without the possibility to control the concentration of a gas (oxygen for example) in a culture liquid, high equilibration times in the culture liquid, high required time in case of switching from a first gaseous concentration to a second gaseous concentration, and the impossibility to create a gradient concentration.

In addition, organs-on-chips are multi-channel 3-D microfluidic cell culture chips that co-culture different types of cells in order to simulate the physiological response of organ systems. Despite the great number of advantages associated with the use of organs-on-chips (such as overcoming problems related to animal experiments, establishing more realistic physiological models than usual cell cultures, avoiding ethical problems, accurately controlling different parameters), organs-on-chips technology is still in its infancy and there are still several technical and industrial challenges to be solved. For example, the accurate control of the oxygen dissolved concentration in cell medium before or while perfusing a microfluidic chip remains a difficult task.

Flow of medium in microfluidic systems is mostly generated either with syringe pumps, peristaltic pumps or with pressure-based controllers. In syringe pumps, the dissolved gas concentration in the fluid contained in the syringe cannot be controlled once the syringe has been filled. Peristaltic pumps induce pulsations and are thus poorly adapted to many applications. Pressure-based controllers are used to control pressure in a special vial in which pressured gas is injected and liquid escapes from a capillary connected to the microfluidic system. In such a system, in order to reach a given liquid flow rate, pressure is changed to accommodate the flowrate read by the flowmeter. Standard pressure controllers typically comprise two valving systems, respectively the admission and emission stages. They can respectively control the injection (i.e admission) or rejection (i.e emission) of input gas in an intermediary cavity to which a pressure sensor is connected. However, these systems present drawbacks regarding the control of dissolved gas species such as long response times when changing the gas composition, or the fact that liquid flow rate and concentration of dissolved species cannot be controlled independently.

Document US 2016/0312166 relates to a method of culturing cells, comprising: disposing cells into a culture chamber, the culture chamber connected to a plurality of microfluidic channels, the culture chamber and microfluidic channels being configured into a culture unit on a well plate; interfacing the well plate with a microincubator manifold, the microincubator manifold having a gasket for providing a removable air-tight seal to the well plate, and a heat exchange module, wherein the manifold seals to the well plate, thereby enclosing an incubation volume of gas, and the heat exchange module provides a controlled temperature above and in communication with the culture chamber.

Document CN 108148749 relates to a micro-fluidic chip culture system for maintaining the dissolved oxygen concentration and acidity (pH) of the culture liquid, characterized in that it keeps the dissolved oxygen concentration of the culture liquid fixed to ensure that the dissolved oxygen concentration of the culture liquid as well as its pH do not change during the cell experiment.

Document KR 101294521 relates to a control device which allows to create hypoxic conditions on a tissue culture-based on microfluidics. This apparatus for controlling the concentration of dissolved oxygen in the cells comprises: a first channel providing space for tissue culture; a second channel disposed on one side of the first channel; an intermediate membrane separating the first and second channels and an oxygen absorbent in the second channel, in order to transfer dissolved oxygen from the first channel to the second through the separation membrane.

Document WO 2019/191685 relates to an automated cell culture system comprising a bioreactor having a plurality of cell culture compartments, the bioreactor having a fluid port fluidly connected to the plurality of cell culture compartments; a pump fluidly connected to the fluid port in order to provide a cell culture medium to the plurality of culture compartments; and a sensor connected to at least one of the plurality of cell culture compartments for measuring at least one cell culture parameter.

Documents US 2013/0295551, WO 2014/082377 and RU 2587628 C1 also relate to other microfluidic devices which modulate or control the gas environment of a cell culture.

The above documents do not make it possible to independently control a dissolved gas concentration and a liquid medium flow rate.

There is thus a need for a device which makes it possible to independently control the concentration of dissolved gas in a liquid medium and the flow rate of the liquid medium while this medium is distributed, e.g. to a cell culture. There is also a need for a device which makes it possible to efficiently and rapidly modify the concentration of dissolved gas in a liquid medium distributed e.g. to a cell culture. Finally, there is a need for a device which provides a liquid medium with a controlled flow rate and with a controlled concentration of a dissolved gas being controlled independently.

## SUMMARY OF THE INVENTION

It is a first object of the invention to provide a device for providing a liquid medium with a controlled flow rate and with a controlled concentration of a dissolved gas, the device comprising:

- a liquid reservoir provided with:

  - at least one gas inlet;
  - at least one gas outlet; and
  - at least one liquid outlet;

- an admission line configured to provide a gaseous medium into the liquid reservoir, the admission line being connected to the at least one gas inlet and comprising at least one control valve; and
- an emission line configured to withdraw the gaseous medium from the liquid reservoir, the emission line being connected to the at least one gas outlet and comprising at least one control valve.

According to some embodiments, the emission line is connected to a pressure controller.

According to some embodiments, the liquid reservoir is provided with at least one inlet configured to receive a sensor, preferably a pressure sensor.

According to some embodiments, the admission line comprises at least one gas flow sensor.

According to some embodiments, the emission line comprises at least one gas flow sensor.

According to some embodiments, the admission line is connected to a single gas source.

According to some embodiments, the admission line branches out and is connected to two or more gas sources containing different gases, each gas source being preferably associated with a dedicated control valve and gas flow sensor.

According to some embodiments, the gaseous medium is air, oxygen, nitrogen or carbon dioxide or a mixture thereof.

According to some embodiments, the gas inlet is configured for bubbling gaseous medium in the liquid medium contained in the liquid reservoir and preferably comprises a tube having a first open extremity and a second open extremity, the second open extremity located in a lower part of the liquid reservoir.

According to some embodiments, the liquid reservoir comprises a liquid agitating element, such as a magnetic stirrer, and mechanical stirrer, an ultrasound or sound transducer, a vibrating element.

A second object of the present invention is to provide an assembly, comprising:

- the device described above; and
- a liquid utilization system comprising at least one liquid inlet and at least one liquid outlet,

wherein the liquid outlet of the device is connected to the liquid inlet of the liquid utilization system.

According to some embodiments, the liquid utilization system is a cell culture system.

According to some embodiments, the assembly further comprises at least one gas sensor for measuring the concentration of a dissolved gas, preferably oxygen or carbon dioxide, in the liquid medium.

According to some embodiments, the assembly comprises an additional reservoir connected to a pressure controller and comprising a liquid inlet connected to the liquid outlet of the liquid utilization system.

According to some embodiments, the assembly comprises a flow restrictor configured for modifying the hydraulic resistance for the liquid medium downstream of the liquid reservoir.

According to some embodiments, the assembly further comprises a recirculation module configured to connect the additional reservoir to the liquid inlet of the liquid utilization system, to the admission line and to the emission line.

According to some embodiments, the liquid utilization system is a microfluidic cell culture system, a millifluidic cell culture system or a nanofluidic cell culture system.

According to some embodiments, the liquid utilization system is devoid of gas channels, gas chambers and gas-permeable materials.

According to some embodiments, the assembly further comprises a control unit configured to receive input from the pressure sensor, the gas flow sensor and/or the liquid flow sensor and/or the gas sensor(s) and to control the control valve of the admission line, the control valve of the emission line, optionally the pressure controller connected to the additional reservoir and/or the flow restrictor, if present, and optionally a regulator on the or each gas source, if present.

Another object of the present invention is to provide a method for providing a liquid medium with a controlled flow rate and with a controlled concentration in a dissolved gas, implemented in the assembly described above, the method comprising the steps of:

- providing the liquid reservoir with a liquid medium;
- flowing gaseous medium into the liquid reservoir via the admission line and out of the liquid reservoir via the emission line, so as to apply a pressure in the liquid reservoir;
- flowing the liquid medium from the liquid reservoir to the liquid utilization system due to the pressure in the liquid reservoir.

According to some embodiments, the method comprises a step of measuring the pressure inside the liquid reservoir.

According to some embodiments, the method comprises a step of measuring the flow rate of the gaseous medium.

According to some embodiments, the method comprises a step of measuring the flow rate of the liquid medium.

According to some embodiments, the method comprises a step of measuring the concentration of one or more dissolved gases, preferably oxygen or carbon dioxide, in the liquid medium.

According to some embodiments, the method comprises a step of measuring the pH of one or more dissolved gases, preferably oxygen or carbon dioxide, in the liquid medium.

According to some embodiments, the method comprises a step of applying a counter-pressure at the liquid outlet of the liquid utilization system.

According to some embodiments, the method comprises a step of providing the gaseous medium from a single gas source.

According to some embodiments, the method comprises a step of providing gases from two or more gas sources and mixing said gases to provide the gaseous medium.

According to some embodiments, the or each gas source is a source of substantially pure oxygen, carbon dioxide, nitrogen or air.

According to some embodiments, the method comprises a step of varying the flow rate of the liquid medium while maintaining the concentration of the dissolved gas in the liquid medium substantially constant.

According to some embodiments, the method comprises a step of maintaining the pressure inside the liquid reservoir constant while modifying the counter-pressure at the liquid outlet of the liquid utilization system.

According to some embodiments, the method comprises a step of modifying the hydraulic resistance downstream of the liquid reservoir.

According to some embodiments, the method comprises a step of modifying the concentration of dissolved gas in the liquid medium, and preferably of modifying the concentration of dissolved oxygen and/or of dissolved carbon dioxide in the liquid medium.

According to some embodiments, the method comprises a step of modifying the pressure in the liquid reservoir.

According to some embodiments, the method comprises a step of modifying the flow rate of gaseous medium flowing through the liquid reservoir.

According to some embodiments, the method comprises a step of modifying the composition of the gaseous medium flowing through the liquid reservoir.

According to some embodiments, the method comprises a step of controlling the control valve(s) on the admission line and/or the control valve on the emission line to adjust the flow rate of the gaseous medium to a predetermined value, preferably using a closed loop regulation.

According to some embodiments, the method comprises a step of controlling the control valve(s) on the admission line and/or the control valve on the emission line to adjust the pressure in the liquid reservoir to a predetermined value, preferably using a closed loop regulation.

According to some embodiments, the method comprises a step of controlling the control valve(s) on the admission line and/or the control valve on the emission line to adjust at least one concentration of dissolved gas, preferably oxygen and/or carbon dioxide, in the liquid medium to a predetermined value, preferably using a closed loop regulation.

According to some embodiments, the method comprises a step of controlling the control valve(s) on the admission line and/or the control valve on the emission line to adjust the pH of the liquid medium to a predetermined value, preferably using a closed loop regulation.

According to some embodiments, the method comprises a step of controlling a counter-pressure at the liquid outlet of the liquid utilization system to adjust the flow rate of the liquid medium from the liquid reservoir to the liquid utilization system, preferably using a closed loop regulation

According to some embodiments, the method comprises controlling a hydraulic resistance downstream of the liquid reservoir to adjust the flow rate of the liquid medium from the liquid reservoir to the liquid utilization system, preferably using a closed loop regulation.

According to some embodiments, both the flow rate of the liquid medium from the liquid reservoir to the liquid utilization system and at least one concentration of dissolved gas, preferably oxygen and/or carbon dioxide, are adjusted independently.

According to some embodiments, the method comprises a step of collecting the liquid medium from the liquid utilization system and flowing it to the additional reservoir.

According to some embodiments, the method further comprises a step of recirculating the liquid medium exiting the liquid utilization system back into the liquid utilization system.

Another object of the present invention is to provide a method of culturing cells in a cell culture system, wherein the liquid utilization system is provided with a liquid medium according to the method described above.

Another object of the present invention is to provide a non-transitory computer readable storage medium having stored thereon instructions that, when executed, cause at least one computing device to control the device described above or the assembly described above so as to carry out the method described above.

The present invention makes it possible to address the need expressed above. In particular, the invention provides a device which makes it possible to independently control the concentration of dissolved gas in a liquid medium and the flow rate of the liquid medium while this medium is distributed, e.g. to a cell culture. In addition, the invention provides a device which makes it possible to efficiently and rapidly modify the concentration of dissolved gas in a liquid medium distributed e.g. to a cell culture. Finally, the invention provides a device which provides a liquid medium with a controlled flow rate and with a controlled concentration of a dissolved gas.

More particularly, the device of the present invention comprises a liquid reservoir, an admission line configured to provide a gaseous medium into the liquid reservoir and an emission line configured to withdraw the gaseous medium from the liquid reservoir. The liquid reservoir is provided with at least one gas inlet connected to the admission line; at least one gas outlet connected to the emission line; and at least one liquid outlet (which makes it possible to distribute the liquid medium to e.g. a cell culture system). The fact that the admission and emission lines are not connected to each other but to a gas inlet and a gas outlet respectively makes it possible to circulate the gaseous medium from the gas inlet into the liquid reservoir and then to the gas outlet in order to promote the exchange of the gaseous medium with the liquid medium and thus to efficiently and rapidly adapt and modulate the concentration of one or more gases in the liquid medium. Input gaseous medium flows into the reservoir before being withdrawn through the emission line. This allows rapid refreshing of the gaseous medium in the reservoir when the gaseous medium is changed. The gas exiting the gas inlet may be bubbled in the liquid medium, or the liquid medium may be agitated as will be explained below.

Furthermore, the concentration of dissolved gas(es) in the liquid medium depends on the composition of the gaseous medium and on the pressure in the liquid reservoir, thus adjusting the pressure or the composition of the gaseous medium allows to control and modify (if necessary) the concentration of one or more dissolved gases in the liquid medium. The pressure, in turn, can be adjusted by controlling the valves of the admission and emission lines. The composition of the gaseous medium can also be modified by independently controlling the valves of the admission line (if there are two or more independent gas sources) or by controlling the gas source itself.

In addition, the device is part of an assembly further comprising a liquid utilization system such as a cell culture system, comprising at least one liquid inlet and at least one liquid outlet. The device and assembly of the present invention make it possible to control the flow rate of the liquid medium in the liquid utilization system by controlling the pressure difference between the liquid inlet and the liquid outlet of the liquid utilization system. In other words, in some preferred embodiments, by e.g. maintaining the pressure at the liquid inlet of the liquid utilization system constant and by modifying the counter-pressure at the liquid outlet of the liquid utilization system, it is possible to adjust the flow rate of the liquid medium independently of (without modifying) the concentration of dissolved gas(es) in the liquid medium. In the case of the present invention, this is made possible without the use of incubators or separate gas and liquid flow elements in the liquid utilization system. Accordingly, the device and assembly of the present invention make it possible to avoid using a channel for gas flow into the liquid utilization system, which simplifies the fabrication process and reduces the fabrication cost.

BRIEF DESCRIPTION OF THE DRAWINGS

**Figure 1** schematically shows an assembly according to one embodiment of the present invention.
**Figure 2** schematically shows a device according to one embodiment of the present invention.
**Figure 3** schematically shows a cap according to one embodiment of the present invention.
**Figure 4** shows a schematic block diagram of an assembly according to one embodiment of the present invention.
**Figure 5** schematically shows an assembly according to one embodiment of the present invention.
**Figure 6A** schematically shows an assembly according to one embodiment of the present invention.
**Figure 6B** schematically shows an assembly according to one embodiment of the present invention.
**Figure 7** shows the experimental isoflow rate curves. The emission ratio $k_e$ can be read on the Y-axis and the admission ratio $k_a$ can be read on the X-axis.
**Figure 8** shows the experimental isopressure curves. The emission ratio $k_e$ can be read on the Y-axis and the admission ratio $k_a$ can be read on the X-axis.
**Figure 9** shows the intersection between the isoflow rate and isopressure curves. The emission ratio $k_e$ can be read on the Y-axis and the admission ratio $k_a$ can be read on the X-axis.
**Figure 10** shows the evolution of the oxygen rate of a liquid medium as a function of time at two different flow rates. The oxygen rate (%) can be read on the Y-axis and the time (sec) can be read on the X-axis.
**Figure 11** shows the evolution of the oxygen rate of a liquid medium as a function of time during a cyclic modification of the oxygen content. The oxygen rate (%) can be read on the Y-axis and the time (sec) can be read on the X-axis.
**Figure 12** shows the variation of the pH as a function of time during bubbling of the liquid medium. The pH can be read on the Y-axis and the time (sec) can be read on the X-axis.

DESCRIPTION OF EMBODIMENTS

The invention will now be described in more detail without limitation in the following description.

Device for providing liquid medium

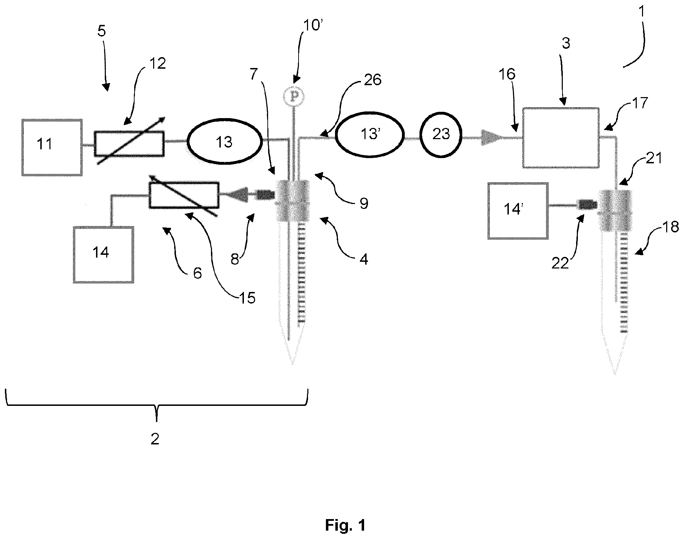

The invention relates to an assembly 1 as illustrated in **figures 1 to 6B** for providing a liquid medium with a controlled flow rate and with a controlled concentration of one or more dissolved gases. By *"concentration in/of dissolved gas"* is meant the quantity of gas dissolved in a given volume of liquid at a constant temperature. According to Henry's law the quantity of gas which dissolves in a given volume of liquid is directly proportional to its partial pressure P in equilibrium with the liquid.

This assembly 1 comprises a device 2 and a liquid utilization system 3 comprising at least one liquid inlet 16 and at least one liquid outlet 17. Thus, the device 2 (which will be further described below) provides a liquid medium to the liquid utilization system 3. This liquid medium is preferably provided at a controlled flow rate and with a controlled concentration of one or more dissolved gases.

In the following, the liquid utilization system 3 is considered to be a cell culture system 3. However, it should be understood that other liquid utilization systems can be used instead of a cell culture system, such as a chemical reactor or a biochemical reactor. As a result, the cell culture system 3 in the following description can be replaced by any other liquid utilization system 3 without modifying the way the assembly operates.

The device 2 according to the invention comprises a liquid reservoir 4 (*i.e.* a reservoir partly filled with liquid medium), an admission line 5 configured to provide a gaseous medium into the reservoir 4, and an emission line 6 configured to withdraw the gaseous medium from the reservoir 4.

The liquid medium is preferably an aqueous solution. In some embodiments, it may be particularly a cell culture medium.

The liquid reservoir 4 is provided with at least one gas inlet 7, at least one gas outlet 8 and at least one liquid outlet 9. The gas inlet 7 of the liquid reservoir 4 is connected to the admission line 5 while the gas outlet 8 of the liquid reservoir 4 is connected to the emission line 6. The liquid outlet 9 of the liquid reservoir 4 is connected to the liquid inlet 16 of the cell culture system 3.

According to some preferred embodiments, the liquid reservoir 4 is provided with a single gas inlet 7.

According to other embodiments, the liquid reservoir 4 is provided with two or more gas inlets 7. In this case, the gas inlets 7 may be connected to different admission lines 5. These different admission lines 5 may for example be used alternatively or jointly. They can be used in a similar manner as will be described below with respect to an admission line 5 having two or more branches.

The or each gas inlet 7 may comprise a tube (or capillary) extending into the liquid reservoir 4. In other words, the gas inlet 7 may comprise a tube having two open extremities, a first open extremity which can be connected to the admission line 5 and a second open extremity located inside the liquid reservoir 4. It is preferable that the second open extremity is located in a lower (bottom) part of the liquid reservoir 4 in order to provide (and thus bubble) the gaseous medium in the liquid medium present in the liquid reservoir 4, even when only a small amount of liquid medium is found into the liquid reservoir 4. In particular, it is preferable that said second extremity is located in the liquid reservoir 4 below the location of the entry point of the gas outlet 8 in said liquid reservoir 4. In other words, the tube of the gas inlet 7 is preferably immersed in the liquid medium. This makes it possible to bubble the gas in the liquid present in the liquid reservoir 4. However, in this case, the device 1 may preferably comprise one or more valves so as to avoid any flow of the liquid (backflow) out of the liquid reservoir 4 through the gas inlet 7 and through the admission line 5.

In some embodiments, the second open extremity may be provided with a nozzle. The presence of the nozzle makes it possible to reduce the size of bubbles gassed into the liquid medium inside the liquid reservoir 4 and thus increase the surface of diffusion between the gas and the liquid medium.

In some other embodiments, the gas inlet 7 may enter the liquid reservoir 4 directly at its lower (bottom) part.

In some other embodiments, the liquid reservoir 4 may comprise a liquid agitating element, such as a magnetic stirrer, and mechanical stirrer, an ultrasound or sound transducer, a vibrating element. This makes it possible to mix the gas entering the liquid reservoir 4 with the liquid present in the liquid reservoir 4 without necessarily bubbling the gas in the liquid.

According to some preferred embodiments, the liquid reservoir 4 is provided with a single gas outlet 8.

According to other embodiments, the liquid reservoir 4 is provided with two or more gas outlets 8. However, it is preferable that the liquid reservoir 4 is provided with only one gas outlet 8.

The gas outlet 8 may comprise a tube (or capillary) extending into the liquid reservoir 4. In other words, the gas outlet 8 may comprise a tube having two open extremities, a first open extremity which can be connected to the emission line 6 and a second open extremity located in the liquid reservoir 4. It is preferable that the second open extremity is located in an upper (top) part of the liquid reservoir 4 in order to withdraw the gaseous medium from the liquid reservoir 4, even when the liquid reservoir 4 is full (or substantially full) of liquid medium. The tube of the gas outlet 8 is not immersed in the liquid medium.

According to some preferred embodiments, the liquid reservoir 4 is provided with a single liquid outlet 9.

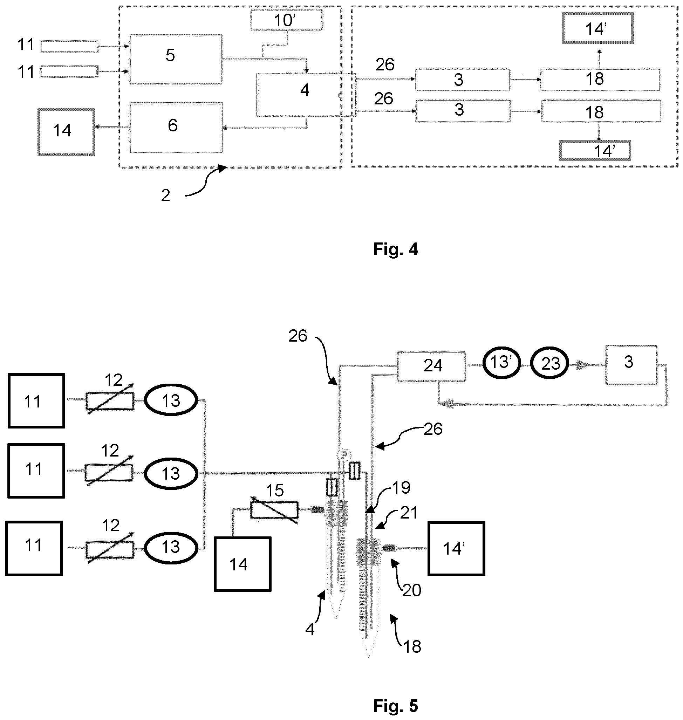

According to other embodiments (as illustrated in **figure 4**), the liquid reservoir 4 is provided with two or more liquid outlets 9. This makes it possible to connect more than one cell culture systems 3 (in parallel for example) to the device 2 according to the invention in order to provide each culture cell system 3 with liquid medium at a controlled flow rate and with a controlled concentration of a dissolved gas. In this case, each cell culture system 3 may be connected to a different liquid outlet 9.

The liquid outlet 9 may comprise a tube (or capillary) extending into the liquid reservoir 4. In other words, the liquid outlet 9 may comprise a tube having two open extremities, a first open extremity which can be connected to the liquid inlet 16 of the cell culture system 3 (for example via a fluidic line 26) and a second open extremity located inside the liquid reservoir 4, so that the tube is immersed in the liquid medium. It is preferable that the second open extremity is located at a lower (bottom) part of the liquid reservoir 4 in order to withdraw liquid from the liquid reservoir 4, even when only a small amount of liquid medium is found into the liquid reservoir 4.

In some embodiments, referred to as *"integrated embodiments"*, the liquid reservoir 4 and the liquid utilization system 3 may be integrated in a single piece, such as a cartridge. In some embodiments, said cartridge can be made from a molded polymer material. In such integrated embodiments, any lines, tubes, or more generally components linking the liquid reservoir 4 and the liquid utilization system 3, can be themselves integrated into the single piece or cartridge. In particular, lines connecting the liquid reservoir 4 to the liquid utilization system 3 may be molded fluidic lines within the single piece or cartridge. A valve may be present at the liquid outlet 9 or on the fluidic line 26 in order to open or close the fluidic connection between the liquid reservoir 4 and the cell culture system 3 (so as to be able to stop the flow of liquid medium).

The liquid reservoir 4 may also preferably comprise at least one inlet 10 configured to receive a sensor 10'. Such sensor 10' may be a pressure sensor, a temperature sensor, a concentration sensor, a pH sensor, a flow sensor, a light sensor, a camera, or a current or voltage sensor. This makes it possible to directly measure a specific property (such as temperature or pressure for example) in the liquid reservoir 4.

According to some embodiments, the liquid reservoir 4 comprises a single inlet 10 configured to receive a sensor. In this case, it is preferable that the sensor is a pressure sensor.

According to other embodiments, the liquid reservoir 4 comprises more than one inlet 10 configured to receive a sensor 10'. In this case, at least one of these inlets 10 is configured to receive a pressure sensor 10'.

Therefore, the device 2 of the present invention may further comprise a sensor 10' chosen, from a pressure sensor, a temperature sensor, a concentration sensor, a flow sensor, a pH sensor, a light sensor, a camera, or a current or voltage sensor, and preferably a pressure sensor, extending into the liquid reservoir 4. The above list must be viewed as a subset of possible sensors, and not as a restriction of the field of the invention, which will also exert its advantages in combination with other types of sensors or detectors.

In some embodiments, a pressure sensor 10' may be present in a location other than the liquid reservoir 4 for example between the admission valve and the emission valve. The pressure sensor 10' may for example be arranged on the admission line 5.

As mentioned above, the admission line 5 is configured to provide the gaseous medium into the reservoir 4. Thus, the admission line 5 may be connected to at least one gas source 11.

According to some embodiments, the or each gas source 11 may be a container 11 containing a pressurized gas (or a gas composition).

The or each gas source 11 may provide a controlled gas composition, a controllable pressure, a controllable flow rate, or any combination thereof. Preferably, the or each gas source 11 provides a predetermined gas composition at a pressure which may be controlled owing to a regulator on the gas source. Alternatively, the gas source 11 may be a gas mixer, or in other words a container comprising at least two pressurized gases (or gas compositions) separated from one another. In this case, a mixture of the separated gases can be provided into the liquid reservoir via the admission line 5. It is preferable that such gas mixer is a Venturi gas mixer, or a tunable gas mixer, so that the composition of the gaseous mixture fed to the admission line 5 can be adjusted by the user.

According to some embodiments (as illustrated in **figure 1**), the admission line 5 may be connected to a single gas source 11.

According to other embodiments, the admission line 5 may be connected to two or more gas sources 11. In particular, the admission line 5 may branch out and be connected to two or more containers 11, each container 11 comprising a different gas or gas composition (and being preferably provided a pressure regulator). In this case, the device 2 may comprise a gas tank between the gas sources 11 and the gas inlet 7, configured to mix the different gases exiting the different gas sources 11 prior to the entrance of the gas mixture into the liquid reservoir 4.

The gas or gas compositions in the gas source(s) 11 may be chosen from air, oxygen, nitrogen, carbon dioxide, carbon monoxide, nitric oxide or hydrogen sulfide. In other embodiments, notably for applications in chemistry, other gases may also be selected *e.g.* among ethylene, carbon monoxide, different nitrogen oxides, ozone, or hydrogen. In case of a single gas source 11, it is preferable that the gas source 11 provides oxygen or air.

In other preferred embodiments, two gas sources 11 independently provide oxygen and carbon dioxide; or oxygen and nitrogen; or nitrogen and carbon dioxide, or air and oxygen; or air and carbon dioxide; or air and nitrogen.

In other preferred embodiments, three gas sources 11 independently provide oxygen, carbon dioxide and nitrogen; or air, carbon dioxide and nitrogen.

Alternatively, a gas mixer may also be used to provide these various gas mixtures.

The admission line 5 comprises at least one control valve 12 (also designated below as an admission valve). Such control valve 12 may be located between the gas source 11 and the gas inlet 7 of the liquid reservoir 4 and can be controlled to adjust the flow rate of the gaseous medium. The control valve 12 may preferably be an electro-valve. In some embodiments, the admission line 5 may also comprise at least one heck valve to prevent backflow of the liquid present in the liquid reservoir 4 in the admission line 5.

An electro-valve is capable of achieving and maintaining different openings depending on the applied voltage.

Alternatively, if the control valve 12 is not an electro-valve, it can be a pneumatically actuated valve, or a mechanically actuated valve.

Optionally, the device 2 may further comprise at least one pressure regulator between the gas source 11 and the control valve 12, configured to regulate the pressure of the gas entering the liquid reservoir 4.

In addition, the admission line 5 may further comprise at least one gas flow sensor 13 in order to measure the flow rate of the gaseous medium. In some other embodiments, temperature of the gas can be controlled by a temperature control system directly integrated in the admission line 5, comprising for instance a Peltier element, or a thermalization fluid circulation, or a resistor, or a temperature sensor, or any combination thereof. Temperature of the gas can also be controlled with an exchanger inside an oven. This allows to equilibrate the liquid source with a gas source at a set temperature (*e.g.* approximately 37°C), otherwise although the liquid source itself is at the set temperature, the liquid in the system could be at a different temperature. This gas flow sensor 13 may be located between the gas source 11 and the gas inlet 7 of the liquid reservoir 4 and preferably between the control valve 12 and the gas inlet 7 of the liquid reservoir 4.

Furthermore, the admission line 5 may comprise one or more than one sensors chosen from a pressure sensor, a temperature sensor, a concentration sensor, a flow sensor, a light sensor, a camera, or a current or voltage sensor.

According to some embodiments, as illustrated in **figure 1**, the device 2 according to the invention comprises a single admission line 5 connected to the gas inlet 7 of the liquid reservoir 4.

According to other embodiments, the device 2 according to the invention may comprise two or more admission lines 5. Thus, each admission line 5 comprises at least one distinct control valve 12 and may be connected to a different gas source 11. This makes it possible to provide the liquid reservoir 4 with different gases.

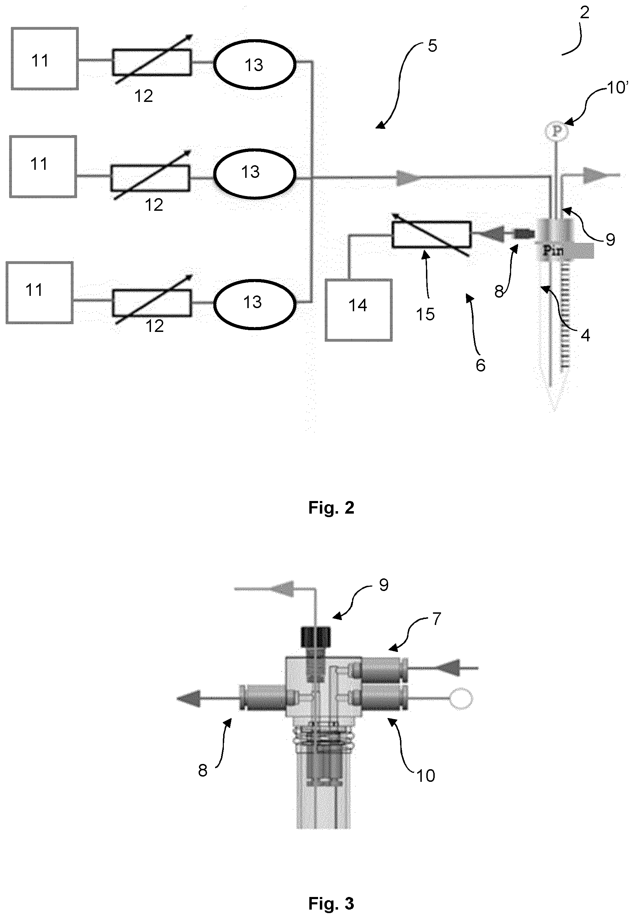

Alternatively, as illustrated in **figure 2**, a single admission line 5 may be used, which branches out and is connected to a plurality of gas sources 11. In this case, preferably, each branch of the admission line 5 comprises a control valve 12 and a gas flow sensor 13. All parallel branches are joined in order to provide a single (mixed) flow of gaseous medium through the gas inlet 7. In this case, the device 2 may comprise an additional gas flow sensor 13 (not illustrated in the figures) downstream of the junction of the branches, in order to monitor (and thus control) the total flow rate of the gaseous medium entering the liquid reservoir 4. Using the individual gas flow sensors 13 and control valves 12 on each branch and optionally the additional gas flow sensor 13 downstream of the junction, it is possible to adjust the composition of the gaseous medium fed to the liquid reservoir 4 as well as its flow rate.

As mentioned above, the emission line 6 is configured to withdraw the gaseous medium from the liquid reservoir 4. The outlet of the emission line 6 may be at the atmosphere. Alternatively, the emission line 6 may be connected to a pressure controller 14 which makes the system independent of variations of atmospheric pressure. This is useful since the concentration of dissolved gas in a liquid medium depends on absolute pressure, not on relative pressure.

By "*pressure controller*" is meant any device (including e.g. valves, pressure sensors and regulators, gas containers and any hydrostatic pressure source) that maintains a pressure at a target absolute value (using *e.g.* an absolute pressure sensor rather than a gauge sensor referenced at the atmospheric pressure).

The emission line 6 comprises at least one control valve 15 (also designated below as an emission valve). Such control valve 15 may be located between the gas outlet 8 of the liquid reservoir 4 and the pressure controller 14 (if present). Alternatively, the control valve 15 may be integrated within the pressure controller 14 itself. The control valve 15 may preferably be an electro-valve, as described above. Alternatively, if the control valve 15 is not an electro-valve, it can be a pneumatically actuated valve, or a mechanically actuated valve.

By controlling the control valve(s) 12 on the admission line 5 and the control valve 15 on the emission line 6, it is possible to adjust the flow rate of gaseous medium into and out of the liquid reservoir 4, and the pressure in the liquid reservoir 4 as will be described in more detail below.

In turn, the pressure inside the liquid reservoir 4 (measured for example with the pressure sensor 10' described above) determines the concentration of dissolved gas(es) in the liquid medium contained in the liquid reservoir 4.

The concentration in a dissolved gas is calculated based on the following equation:

$$[\text{dissolved gas component}] = K_h \text{ x proportion of gas component in gaseous medium x P}$$

$K_h$ being Henry's constant and P being the total pressure in the liquid reservoir.

Furthermore, the emission line 6 may comprise one or more than one sensors chosen from a pressure sensor, a temperature sensor, a concentration sensor, a flow sensor, a light sensor, a camera, or a current or voltage sensor.

For example, the emission line 6 may comprise at least one flow sensor (not illustrated in the figures) in order to measure the flow rate of the gaseous medium withdrawn from liquid reservoir 4. This flow sensor may be located between the gas outlet 8 of the liquid reservoir 4 and the pressure controller 14. This may be in addition to or instead of the flow sensor on the admission line 5.

The device 2 according to the invention may comprise one or more emission lines 6 connected to the gas outlet 8 of the liquid reservoir 4. Preferably, it comprises a single emission line 6.

According to some embodiments, the liquid reservoir 4 is a one-piece container comprising at least one gas inlet 7, at least one gas outlet 8, at least one liquid outlet 9 and optionally the at least one inlet 10 configured to receive a sensor.

According to other preferred embodiments, the liquid reservoir 4 is a multiple-piece container. Preferably, the liquid reservoir 4 comprises a cap and a vial, the cap and the vial being assembled in order to form the liquid reservoir 4. For example, the cap can be screwed or clipped on the vial to form the liquid reservoir 4.

When the liquid reservoir 4 is a multiple-piece container, the at least one gas inlet 7, at least one gas outlet 8, at least one liquid outlet 9 and optionally the at least one inlet 10 configured to receive a sensor 10' are preferably located on the cap. Such cap is illustrated in **figure 3.**

The vial may be a common conical tube used for cell culture which can be connected to the cap. It can be any vial able of containing a liquid medium. Such vial can be made from materials such as polypropylene, polystyrene, polyethylene, glass, or polycarbonate, polypropylene, cyclic olefin polymers or copolymers, polyether ether ketone (PEEK), perfluorocarbons or fluorocarbons, acrylic polymers, or more generally thermoplastic materials and polymers, thermosetting resins such as epoxys.

According to some embodiments, when the liquid reservoir 4 is a multiple-piece container, the liquid reservoir 4 may also comprise a sealing member able to provide gas-tightness between the cap and the vial. Such member may be made from a material chosen from metal, polypropylene, polycarbonate, polystyrene polypropylene, cyclic olefin polymers or copolymers, PEEK, perfluorocarbons or fluorocarbons, or more generally thermoplastic materials and polymers. Particularly, said sealing member may be an elastic material, such as elastomers, siloxane elastomers, polydimethylsiloxane (PDMS), natural rubber, polyenes such as notably polyisoprene and its copolymers, polybutadiene, thermoplastic elastomers, fluorinated elastomers, polyurethanes, polyacrylates, epoxys.

The sealing member may for example be an O-ring, an elastomer gasket or any deformable part. The sealing member may be squeezed between the cap and the vial.

The device 2 according to the present invention is preferably a transportable device. It may have a weight from 1 mg to 10 kg and preferably from 100 g to 5 kg.

It may also have a volume from 10 cm$^3$ to 1 L and preferably from 50 cm$^3$ to 500 $_{cm}$$^3$.

When the liquid reservoir 4 is a multiple-piece container, the vial may have a volume from 10 $\mu$L to 1 L, and preferably from 1 mL to 100 mL, and more preferably from 1 mL to 15 mL.

Assembly comprising the above device and a liquid utilization system

As mentioned above, the assembly 1 according to the present invention also comprises a liquid utilization system 3 such as a cell culture system 3 having a liquid inlet 16 and a liquid outlet 17. The device 2 according to the present invention is connected to a cell culture system 3, and more particularly, the liquid outlet 9 of the device 2 according to the present invention is connected to the liquid inlet 16 of the cell culture system 3. In some embodiments, the device 2 and the liquid utilization system 3 are integrated as a single component, such as a cartridge. Otherwise, the liquid utilization system 3 is a component separate from the device 2, and fluidically connected to it by e.g. tubing.

By" *cell culture system"* is meant a system in which cells are loaded and cultured. The cell culture system 3 comprises at least one channel through which the liquid medium provided by the above-mentioned device 2 can flow. According to some embodiments, the cell culture system 3 may comprise more than one channels. The cell culture system 3 of the present invention is preferably gas-tight. Thus, the cell culture system 3 should be made of a material which is substantially non-permeable to gases. Such material may be chosen from poly(methyl methacrylate) (PMMA), metal, polyproprylene, polycarbonate, polystyrene polypropylene, cyclic olefin polymers or copolymers (COC), PEEK, perfluorocarbons or fluorocarbons, or more generally thermoplastic materials and polymers. Particularly, said member may be an elastic material, such as elastomers, siloxane elastomers, PDMS, natural rubber, polyenes such as notably polyisoprene and its copolymers, polybutadiene, thermoplastic elastomers, fluorinated elastomers, polyurethanes, polyacrylates, epoxys.

The cell culture system 3 may be chosen from a millifluidic cell culture system, or a microfluidic cell culture system, or a nanofluidic cell culture system. Preferably, it is a microfluidic cell culture system.

By *"millifluidic cell culture system"* is meant a cell culture system in which the minimal channel or chamber dimensions are of the order of 1-10 mm.

By *"microfluidic cell culture system"* is meant a cell culture system in which the minimal channel or chamber dimensions are of the order of 1 to less than 1000 $\mu$m.

By *"nanofluidic cell culture system"* is meant a cell culture system in which the minimal channel or chamber dimensions are of the order of less than 1 $\mu$m.

In the following text and claims, only the term *"microfluidic"*, and in some instances *"microfluidic chip"* are used for convenience, but such terms also encompass millifluidic and nanofluidic systems. Also, these terms encompass any systems comprising channels with the dimensions stated above, whether made by microfabrication, or by the assembly of microcapillaries, or more generally by any method able to provide systems with channels of the specified dimensions. Also, they are not restricted to two-dimensional *"chips"*, and encompass any 1D, 2D or 3D geometries of such channels, chambers, or arrays of channels or chambers or any combination thereof

According to some embodiments, the cell culture system 3 of the present invention may be an organ-on-chip or a tissue-on-chip. By "*organ-on-a-chip*" or by "*tissue-on-a-chip*" is meant a multi-channel 3-D microfluidic cell culture chip that simulates the activities, mechanics and physiological response of entire organs, organ systems or tissues.

The cell culture system 3 is preferably devoid of separate gas flow elements. Thus, the cell culture system 3 may be devoid of a gas channel, or a gas chamber or a gas-permeable material (such as a membrane). This makes it possible to reduce fabrication cost and simplify the fabrication process.

According to some embodiments, the cell culture system 3 comprises a single liquid inlet 16. In this case, the liquid outlet 9 of the liquid reservoir 4 is connected to the liquid inlet 16 of the cell culture system 3 (for example via a fluidic line) in order to provide the at least one channel of the cell culture system 3 with liquid medium having a controlled flow rate and a controlled concentration in a dissolved gas.

According to other embodiments, the cell culture system 3 comprises two or more liquid inlets 16. In this case, the liquid outlet 9 of the liquid reservoir 4 may be connected to all liquid inlets 16 of the cell culture system 3 (for example in order to provide the two or more channels of the cell culture system 3 with the liquid medium having a controlled flow rate and a controlled concentration in a dissolved gas), or each liquid inlet 16 of the cell culture system 3 may be connected to a different liquid outlet 9 of the same liquid reservoir 4 (still in order to provide each channel of the cell culture system 3 with the liquid medium having a controlled flow rate and a controlled concentration in a dissolved gas).

According to other embodiments, the assembly 1 may comprise a single device 2 connected via a plurality of liquid outlets 9 of the liquid reservoir 4, to multiple cell culture systems 3, each cell culture system 3 being connected to a different liquid outlet 9 of the liquid reservoir 4.

According to other embodiments, the assembly 1 may comprise a single cell culture system 3 connected to two or more devices 2, in particular in order to provide different channels of the cell culture system 3 with different liquid media.

According to other embodiments, the assembly 1 may comprise multiple cell culture systems 3 connected to multiple devices 2, in particular in order to provide different channels of the various cell culture systems 3 with different liquid media.

The assembly 1 can further comprise one or more sensors connected to the cell culture system 3 such as a pressure sensor, a temperature sensor, a concentration sensor, a pH sensor, a flow sensor, a light sensor, a camera, or a current or voltage sensor.

Preferably, the cell culture system 3 may be connected to a liquid flow sensor 13' in order to measure the flow rate of the liquid medium into the cell culture system 3. Such sensor may be for example located between the liquid outlet 9 of the liquid reservoir 4 and the liquid inlet 16 of the cell culture system 3 (although it could also be placed downstream of the cell culture system 3).

In addition, as illustrated in **figure 1**, the assembly 1 according to the invention may further comprise a dissolved gas sensor 23 in order to measure the concentration in dissolved gas entering the cell culture system 3. The dissolved gas sensor 23 can be located between the liquid outlet 9 of the liquid reservoir 4 and the liquid inlet 16 of the cell culture system, and on the fluidic line 26, for instance between the liquid flow sensor 13' (if such sensor is present) and the liquid inlet 16 of the cell culture system 3.

The dissolved gas sensor 23 may be for example an oxygen sensor, or a nitrogen sensor, or a carbon dioxide sensor. More than one dissolved gas sensors may be used in order to accurately characterize a complex dissolved gas mixture, for instance an oxygen sensor and/or a nitrogen sensor and/or a carbon dioxide sensor.

The liquid outlet 17 of the cell culture system 3 may be at ambient (atmospheric) pressure. Alternatively, the pressure at the liquid outlet 17 of the cell culture system 3 may be controlled owing to an outlet gas pressure source which may be the same as the outlet pressure source of the device 2 or which may be different. The set pressure may be below or above atmospheric pressure.

One way to provide an outlet gas pressure source is to provide an additional reservoir 18.

When the device 2 is connected to more than one cell culture systems 3 in parallel as illustrated in **figure 4**, one additional reservoir 18 may be associated with each cell culture system 3.

The or each additional reservoir 18, as illustrated in **figure 1**, may comprise at least one liquid inlet 21 and be further connected to a pressure controller 14' *via* a gas connection 22. The liquid outlet 17 of the cell culture system 3 may be connected to the liquid inlet 21 of the additional reservoir 18. Thus, the liquid medium may exit the cell culture system 3 and enter the additional reservoir 18. The pressure controller 14' makes it possible to impose a certain pressure within the additional reservoir 18.

Alternatively, the additional reservoir 18, as illustrated in **figure 5**, may comprise at least one liquid inlet 21, at least one gas inlet 19 and at least one gas outlet 20. In this case, the configuration of the additional reservoir 18 be similar to the that of the liquid reservoir 4 described above (with the difference that the additional reservoir 18 comprises at least one liquid inlet 21 while the liquid reservoir 4 comprises at least one liquid outlet 9). The liquid outlet 17 of the cell culture system 3 may be connected to the liquid inlet 21 of the additional reservoir 18. Thus, the liquid medium may exit the cell culture system 3 and enter the additional reservoir 18.

The gas inlet 19 of the additional reservoir 18 may be connected to the admission line 5.

The gas outlet 20 of the additional reservoir 18 may be connected to a pressure controller 14' which makes it possible to impose a certain pressure at the gas outlet 20.

This configuration is especially useful when the assembly comprises a recirculation capability, as will be further described below.

According to some embodiments, in case several cell culture systems 3 or several channels in a single cell culture system 3 are provided with liquid medium from a single device 2 according to the invention, these several cell culture systems 3 or these several channels may be connected to the same additional reservoir 18.

According to other embodiments, in case several cell culture systems 3 or several channels in a single cell culture system 3 are provided liquid medium from a single device 2 according to the invention, these several cell culture systems 3 or these several channels may be connected to different additional reservoirs 18.

Alternatively to the pressure controller 14' or additionally, the assembly 1 according to the present invention may comprise a flow restrictor (not illustrated in the figures). According to some embodiments, more than one flow restrictors can be comprised in the assembly 1. A flow restrictor may be placed upstream of, within, or downstream of the cell culture system 3. According to preferred embodiments, the liquid inlet 16 of the cell culture system 3 may be connected to a flow restrictor. The flow restrictor (which can be for example, and non limitatively, a pinch valve, or a solenoid valve, or a piezo valve, or a passive capillary, or a mechanical valve, or an electro-valve or a filter, preferably a pinch valve) can be located between the liquid outlet 9 of the liquid reservoir 4 and the liquid inlet 16 of the cell culture system 3, and particularly between the liquid flow sensor 13' (if such sensor is present) and the liquid inlet 16 of the cell culture system 3. The flow restrictor allows to modify the hydraulic resistance, therefore modifying the liquid flow rate without modifying the concentration of dissolved gas(es) in the liquid medium.

As illustrated in **figure 5**, the assembly 1 according to the invention may further comprise a recirculation module 24. The recirculation module 24 is configured to connect the additional reservoir 18 to the liquid inlet 16 of the cell culture system 3, to the admission line 5 and to the emission line 6. The recirculation module 24 may switch between the configuration described above, and another configuration wherein the additional reservoir 18 plays the role of the liquid reservoir 4, and the liquid reservoir 4 plays the role of the additional reservoir 18.

More specifically, the recirculation module 24 allows to switch the function of the liquid outlet 9 of the liquid reservoir 4 to a liquid inlet 9 and to switch the function of the liquid inlet 21 of the additional reservoir 18 to a liquid outlet 21. As a result, the liquid medium comprised in the additional reservoir 18 can recirculate into the cell culture system 3 from the liquid inlet 16 to the liquid outlet 17 and can then enter the liquid reservoir 4. This is useful in particular in order to continue to feed the cell culture system 3 with liquid medium after the amount of liquid medium in the liquid reservoir 4 has become relatively low. This has many technical advantages. For instance, it may reduce medium consumption, and require less manual operation. It may also allow to preserve in the medium, products secreted by cells, and thus improve homeostasis and avoid brutal changes in the environment of cells.

The recirculation module 24 may comprise one or more than one valves to control liquid and gas flow to and from the cell culture system 3, the liquid reservoir 4 and the additional reservoir 18. Preferably, the recirculation module 24 may comprise more than one valves, preferably two or more valves, preferably three or more valves, and more preferably four or more valves.

The recirculation module may comprise one or more than one pressure controllers. Preferably, the recirculation module may comprise more than one pressure controller, and preferably two or more pressure controllers.

In some embodiments, the recirculation module 24 may comprise an input reservoir selection valving stage to select if the gaseous medium flows to one reservoir or the other; an output reservoir selection valving stage to select if the emission valve receives gaseous medium from one reservoir or the other; a liquid redirection valving stage to select the direction of flow of the liquid medium between the reservoirs.

In some embodiments, the recirculation module 24 additionally comprises a three-way valve, so as to switch between a first mode in which the cell culture system 3 is filled, and its liquid outlet 17 is connected to a waste reservoir, and a second recirculation mode, in which said liquid outlet 17 is connected to the liquid reservoir 4.

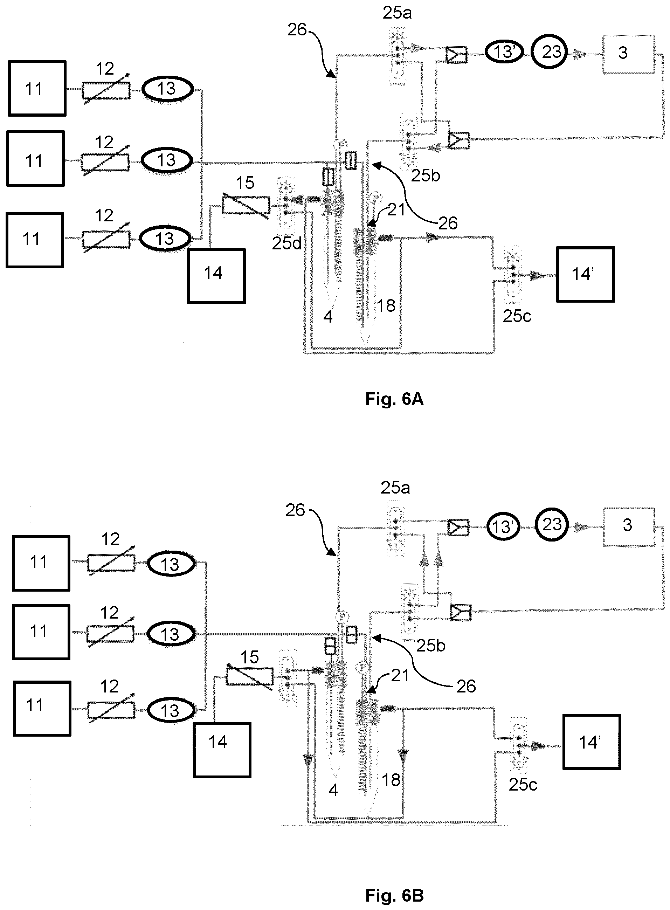

An example of recirculation module 24 is better illustrated in **figures 6A** and **6B.** Valves 25a, 25b, 25c and 25d are part of the recirculation module 24.

First valve 25a and second valve 25b are responsible for controlling the liquid flow between cell culture system 3, the liquid reservoir 4 and the additional reservoir 18.

Third valve 25c and fourth valve 25d are responsible for controlling the gas flow between the liquid reservoir 4 or the additional reservoir 18 and the emission line 6 or the pressure controller 14.

In more detail, according to the configuration illustrated in **figure 6A**, the liquid medium flows out of the liquid outlet 9 of the liquid reservoir 4, through first valve 25a and into the cell culture system 3. At this stage, the emission line 6 is connected to the liquid reservoir 4 due to the configuration of the fourth valve 25d. Then the liquid medium exiting the cell culture system 3 flows through second valve 25b and then enters the additional reservoir 18 via the liquid inlet 21. At this stage, the pressure controller 14' is connected to the additional reservoir 18 due to the configuration of the third valve 25c. At a later stage, for example when a large portion of the liquid medium has been transferred from the liquid reservoir 4 to the additional reservoir 18, the valves can be switched as illustrated in **figure 6B**. Then, the liquid outlet 9 becomes a liquid inlet 9 while the liquid inlet 21 becomes a liquid outlet 21.

As illustrated in **figure 6B**, the liquid medium flows out of the liquid outlet 21 of the additional reservoir 18, through the second valve 25b, and into the cell culture system 3. At this stage, the emission line 6 is connected to the additional reservoir 18 due to the configuration of the fourth valve 25d. Then the liquid medium exiting the cell culture system 3 flows through the first valve 25a and enters the liquid reservoir 4 via the liquid inlet 9. At this stage, the pressure controller 14' is connected to the liquid reservoir 4 due to the configuration of the third valve 25c.

According to other embodiments (not illustrated in the figures), the recirculation can be achieved by a liquid recirculation pump allowing to feed back the liquid medium exiting the liquid outlet 17 of the cell culture system 3 into the liquid reservoir 4. To implement this, the recirculation module may comprise an additional fluidic connection between the liquid outlet 17 of the cell culture system 3 and the liquid reservoir 4, and a liquid recirculation pump, placed either along the fluidic line 26 between the liquid reservoir 4 and the cell culture system 3, or within the cell culture system 3, or along a second fluidic line connecting the liquid outlet 17 of the cell culture system 3 to the liquid reservoir 4.

According to preferred embodiments, the assembly 1 according to the invention, is devoid of an incubator.

The cell culture system 3 may be placed in an oven, so that it remains at a controlled temperature, for instance a constant temperature of approximately 37°C. The oven may be part of the assembly 1. In some other embodiments (and as described above), temperatures can be controlled by a temperature control system directly integrated in the microfluidic device, comprising for instance a Peltier element, or a thermalization fluid circulation, or a resistor, or a temperature sensor, or any combination thereof.

Control of the device and assembly of the invention

The assembly 1 according to the invention may be connected to or may comprise a control unit. The control unit makes it possible for a user to control one or more than one parameters, such gas flow rate, liquid flow rate, dissolved gas concentration, pressure... The control unit may comprise a graphical user interface which allows to choose an input value for one or more than one parameters.

The control unit may ensure a fully automated operation of the device 2 and assembly 1.

The control unit may comprise one or more processors coupled to a storage medium, as well as a computer program comprising instructions stored thereon, for performing various steps described in more detail below. The control unit may receive input from any combination of the sensors mentioned above, as well as input from the user. In particular, the control unit may receive input from one or more of: the pressure sensor 10' associated with the liquid reservoir 4, the gas flow sensor(s) 13 on the admission line 5, the gas flow sensor(s) 13 on the emission line 6, the liquid flow sensor 13' and optionally the dissolved gas sensor(s) 23 for the liquid medium (positioned e.g. on the fluidic line 26). The control unit may also receive input from any other pressure sensor, flow sensor, light sensor, pH sensor, camera, current or voltage sensor which may be present in the assembly 1.

The control unit may process the input data and/or the user instructions and as a result, provide instructions to the various control valves and pressure controllers, and in particular to the control valve(s) 12 on the admission line 5, the control valve(s) 15 on the emission line 6 and the pressure controller 14 connected to the emission line 6, if present. It may also provide instructions to the pressure controller 14' connected to the additional reservoir 18 (if present) and/or to the flow restrictor (if present).

The control unit may in particular control the aperture ratios of the various control valves.

The aperture ratio k of each control valve can be defined as a ratio of the gas flow rate Q through the valve to the maximum gas flow rate $Q_{max}$ which can be achieved. As a result, when the valve is fully closed, k=0 and when it is fully open, k=1.

If the admission valve 12 is closed ($k_a$=0) or if the emission valve 15 is closed ($k_e$=0), the gas flow rate is zero. If both of the valves are completely open ($k_a$=$k_e$=1), the flow rate is maximal (Q=$Q_{max}$). Changing the opening ratio of the two valves allows to obtain different flow rates. The theoretical approximative relationship between the admission ratio ($k_a$), the emission ratio ($k_e$) and the gas flow rate (Q), at steady state and assuming the gas flow is significantly higher than the liquid flowing out of the reservoir 4, is the following:

$$k_e = \frac{k_a * Q}{k_a * Qmax - Q}$$

Therefore, controlling the admission valve 12 and the emission valve 15 makes it possible to adjust the flow rate of gaseous medium through the liquid reservoir 4.

Similar considerations apply when two or more admission lines are present.

The aperture ratios also make it possible to control the pressure P in the liquid reservoir 4. If the admission valve 12 is closed and the emission valve 15 is open, P is equal to the outlet pressure of the emission valve 15 $Pout_{gas}$ (atmospheric pressure or pressure set by the pressure controller 14). If the admission valve 12 is open and the emission valve 15 is closed, P is equal to $P_{max}$, namely the input gas pressure set by the gas source 11. If both valves are open, the theoretical relationship is the following:

$$k_e = k_a * \left(\frac{Pmax - Pout_{gas}}{P - Pout_{gas}} - 1\right)$$

In this equation, $P_{max}$ is the input gas pressure (set by the gas source 11), $Pout_{gas}$ is the outlet pressure of the emission valve 15 (atmospheric pressure or pressure set by the pressure controller 14). In some embodiments, $P_{max}$ may be set by the user or controlled by the control unit by adjusting a regulator or a pump on the or each gas source 11.

As a result, by controlling both the admission valve 12 and the emission valve 15, it is possible to adjust the flow rate of gas Q through the liquid reservoir 4, as well as the pressure P inside the liquid reservoir 4.

As a reminder, the concentration of dissolved gas in the liquid medium varies depending on the pressure P in the liquid reservoir 4.

Besides, the flow rate of liquid medium exiting the liquid reservoir 4 depends on the difference between the pressure P in the liquid reservoir 4 and the pressure $Pout_{liq}$ at the outlet of the cell culture system 3 (also designated as *"counter-pressure"*), which in some embodiments can be adjusted by the associated pressure controller 14'.

In summary, by controlling the admission valve 12, the emission valve 15 and pressure controller 14' downstream of the cell culture system 3, it is possible to independently control the concentration of gas dissolved in the liquid medium (which depends on P) and the liquid flow rate to and through the cell culture system 3 (which depends on P - $Pout_{liq}$).

By way of example, by maintaining the pressure P in the liquid reservoir 4 constant, and by modifying the counter-pressure at the liquid outlet 17 of the cell culture system 3, the flow rate of the liquid medium can be varied without modifying the dissolved gas concentration in the liquid medium.

In some embodiments, the pressure controller 14' downstream of the cell culture system 3 can be set to a pressure which may be higher or lower than atmospheric pressure. Using a pressure lower than atmospheric pressure is useful for achieving higher liquid flow rates. Using a pressure higher than atmospheric pressure may be useful to avoid the formation of bubbles in the cell culture system.

In other embodiments, the flow restrictor (such as pinch valve) described above is used to adjust and modify the hydraulic resistance R of the system. Therefore, the flow rate of liquid medium $Q_{liq}$ for a given pressure P in the liquid reservoir 4 may be adjusted even without modifying the counter-pressure $Pout_{liq}$, based on the theoretical relationship $(P-Pout_{liq}) = Q_{liq} \times R$. This may make it possible to simply connect the liquid outlet 17 of the cell culture system 3 to atmospheric pressure, instead of a pressure controller 14', and still achieve a control over the flow rate of the liquid medium.

Experimentally, deviations are typically observed relative to theoretical relationships described above.

Therefore, advantageously, a regulation algorithm is implemented in the control unit so as to adjust the control of the valves, pressure controllers and/or flow restrictor as a function of input from the various sensors (in particular the pressure sensor, liquid flow rate sensor and gas sensor). In some preferred embodiments, said regulation algorithm involves a closed loop configuration. The regulation algorithm may be of the proportional type (P), integral type (I), derivative type (D), proportional-integral type (PI), proportional-derivative type (PD), integral-derivative type (ID) or preferably proportional-integral-derivative type (PID).

In some embodiments, the regulation algorithm may rely on fuzzy logic, artificial intelligence, deep learning or neural networks, to optimize the regulation.

In some embodiments, one or more target values are input by the user or otherwise predetermined, such as a gas flow rate and/or a liquid flow rate and/or one or more concentration of dissolved gas in the liquid medium, and the control unit, using the regulation algorithm, and based on inputs from one or more sensors as described above, sends instructions to the control valves, pressure controllers and/or flow restrictor in order to reach the target values as quickly as possible (or within a predetermined timeframe). As an initial phase of the regulation, the control valves, pressure controllers and/or flow restrictor may be controlled based on preset values which may be derived from the theoretical relationships described above, or from an experimental calibration of the device, before starting the closed loop regulation per se.

By way of example, the regulation algorithm may include the following steps, in order to adjust the gas flow rate and the pressure in the liquid reservoir 4 to target values (which may be set by the user):

- Open the admission valve and emission valve according to a predefined table, depending on the gas flow rate setpoint.
- Read the sensed gas flow rate.
- Launch a closed loop (*e.g.* PID) regulation on the admission valve opening in order to reach and maintain the flow rate at or near the desired setpoint.
- Read the sensed pressure.
- Launch a closed loop (*e.g.* PID) regulation on the emission valve opening in order to reach and maintain the pressure at or near the desired setpoint.

Hence, the gaseous flow rate and the pressure are regulated independently, but consecutively in the same program loop.

As another example, when more than one gas sources 11 is present (as illustrated in **figure 2**), the regulation algorithm may include the following steps, in order to adjust the individual gas flow rates and the pressure in the liquid reservoir 4 to target values (which may be set by the user):

- Open the admission valve and emission valve according to a predefined table, depending on the setpoints.
- Read each sensed gas flow rate and launch a closed loop (*e.g.* PID) regulation on the corresponding admission valve opening in order to reach and maintain the flow rate at or near the desired setpoint.

- Read the sensed pressure.
- Launch a closed loop (*e.g.* PID) regulation on the emission valve opening in order to reach and maintain the pressure at or near the desired setpoint.

As a variation, the user may for instance input the total gas flow rate as well as the composition of the gas flow, and the control unit determines the setpoint individual gas flow rates required to reach these targets. For example, if three gas containers 11 containing oxygen (or air), nitrogen and carbon dioxide are used, the user may select a target total gas flow rate, and two target gas concentrations in the liquid medium (for example oxygen and nitrogen, or nitrogen and carbon dioxide, or oxygen and carbon dioxide). The control unit then determines the individual gas flow rates required to reach these three target values, and then implements the above regulation algorithm.

In some preferred embodiments, the control unit may use information gained from the analysis of images taken by a camera in its regulation action. In particular, said images may provide from imaging of the cell culture system 3, or even preferably from imaging of the cells contained in said cell culture system 3. Said analysis may provide information about the number, the shape, the metabolism, or other properties of the cells. Thus, the result of an image analysis may be used within the invention in combination or instead of the output of a "*sensor*" whenever such input is mentioned in the description of the invention.

Use of the device and assembly

The invention further relates to a method for providing a liquid medium with a controlled flow rate and with a controlled concentration in a dissolved gas. Preferably, this method is implemented in the assembly 1 described above.

The method according to the invention comprises a first step of providing the liquid reservoir 4 with a liquid medium. The *"liquid medium"* is preferably a *"growth medium", i.e.* an aqueous composition adapted to support the growth of a population of microorganisms or cells via the process of cell proliferation.

Once the liquid medium is in the liquid reservoir 4, the method comprises a step of feeding, preferably in a continuous manner (but also possibly in an intermittent manner), at least one gas from a gas source 11 into the liquid reservoir 4 via the admission line 5 and continuously withdrawing the gas from the liquid reservoir 4 via the emission line 6. The gas flow rate may for example be from 2 mL/min to 5 L/min.

According to some preferred embodiments, the gas flow rate may be from 50 mL/min to 700 mL/min.

According to other preferred embodiments, the gas flow rate may be from 2 mL/min to 50 mL/min.

According to other preferred embodiments, the gas flow rate may be from 500 mL/min to 5 L/min.

The gas flow rate can be controlled (imposed) by the control valves 12, 15 of the admission line 5 and emission line 6 as described above. The gas is brought in contact with the liquid medium. The concentration in dissolved gas in the liquid medium can be controlled owing to the pressure in the liquid reservoir 4 as described above.

The method according to the invention also comprises a step of flowing the liquid medium from the liquid reservoir 4 and to the cell culture system 3. The flow rate of the liquid medium can be adjusted depending on the pressure in the liquid reservoir 4 and the counter-pressure at the liquid outlet 17 of the cell culture system 3. The liquid medium flow rate may be for example from 0.5 $\mu$L/min to 200 $\mu$L/min (in particular for organ-on-chip applications) or 200 $\mu$L/min to 50 mL/min or 50 to 100mL/min.

Preferably, the liquid medium enters the cell culture system 3 from the liquid inlet 16 and is withdrawn from the liquid outlet 17 and fed to the additional reservoir 18, if present.

The method according to the invention makes it possible to control and adjust the liquid flow rate of the liquid medium in the cell culture system 3 (independently from the dissolved gas concentration in the liquid medium).

In some variations, the method comprises a step of flowing the gaseous medium so as to reach a target dissolved gas concentration in the liquid medium, without any flow of liquid medium, and then a second step of flowing the liquid medium having the target dissolved gas concentration. The transition from the first step to the second step can be achieved by opening a valve between the liquid reservoir 4 and the cell culture system 3. Advantageously, the gas keeps flowing during the second step to ensure that the dissolved gas concentration in the liquid medium remains at the targeted level. In some variations, the gas flow rate in the first step is higher than the gas flow rate in the second step. Therefore, the target dissolved gas concentration may be relatively quickly achieved in the first step, and then the gas flow rate may be lowered to a level which is sufficient to maintain the target dissolved gas concentration and to reduce the consumption of the gas in the gas container at the inlet. By way of example, the gas flow rate in the first step may be from 150 mL/min to 700 mL/min, and the gas flow rate in the second step may be from 1 mL/min to 300 mL/min.

In some embodiments, the method comprises keeping the dissolved gas concentration in the liquid medium substantially constant while varying the flow rate of liquid medium. This can be performed by maintaining the pressure inside the liquid reservoir 4 at a substantially constant level, and by varying the counter-pressure applied at the liquid outlet 17 of the cell culture system 3. This counter-pressure may be adjusted owing to the pressure controller 14' which may be connected to the additional reservoir 18.

The difference between the pressure in the liquid reservoir 4 and the counter-pressure may for example range from 10 mbar to 7800 mbar, and preferably from 50mbar to 1000 mbar.

Alternatively, this can be performed by maintaining the pressure inside the liquid reservoir 4 at a substantially constant level, and by varying the hydraulic resistance of the system (for example by using a flow restrictor as detailed above). In this case, the counter-pressure at the liquid outlet 17 of the cell culture system 3 can be kept substantially constant.

It is also possible to modify both the hydraulic resistance and the counter-pressure.

Alternatively, the method may comprise varying the concentration of the dissolved gas in the liquid medium, either while keeping the liquid flow rate substantially constant, or while varying the liquid flow rate.

This can be performed notably by modifying the pressure in the liquid reservoir 4. By simultaneously adjusting if necessary the counter-pressure and/or the hydraulic resistance, the liquid flow rate may be kept substantially constant despite the modification of the pressure in the liquid reservoir 4, or may be adjusted as needed.

In some preferred embodiments, the concentration of dissolved oxygen in the liquid medium is varied as described above. This makes it possible to culture cells under different conditions representative of *in vivo* conditions in various healthy or diseased (*e.g.* tumoral) tissues.

In some embodiments, the concentration of dissolved gas may be varied in an intermittent or cyclic manner. This makes it possible to culture cells for instance under conditions representative of intermittent hypoxia as can be found in sleep apnea and myocardial infarction.

When more than one gas sources are used, the concentration of the dissolved gases in the liquid medium may also be modified (instead of, or in addition to modifying the pressure in the liquid reservoir 4), by modifying the composition of the gaseous medium flowing to the liquid reservoir 4. This can be performed by individually controlling the control valves 12 associated with the various gas sources; or by switching between different gas sources comprising different gas compositions.

In some embodiments, the gaseous medium comprises oxygen and carbon dioxide, the proportion of which may be independently controlled as described above (using at least two separate gas sources). This makes it possible to independently control the concentration of dissolved oxygen and dissolved carbon dioxide in the liquid medium. The control of the concentration of dissolved carbon dioxide, in turn, makes it possible to control and adjust the pH of the liquid medium (for example at or close the normal physiological value of 7.4, or at or close a different value, such as less than 7.4, in order to simulate a disease condition).

Furthermore, the method according to the present invention may comprise a step of recirculating the liquid medium exiting the cell culture system 3 (from the liquid outlet 17) and entering the additional reservoir 18, into the cell culture system 3 (by using for example the recirculation module 24 described above). More specifically, as explained above (and as illustrated in **figures 6A** and **6B**), when a major portion of the liquid medium has been transferred from the liquid reservoir 4 to the additional reservoir 18 and due to the presence of the recirculation module 24, the method according to the present invention may comprise the steps of continuously providing the at least one gas into the additional reservoir 18 via the admission line 5 (in the same way as explained above in the case of the liquid reservoir 4), continuously withdrawing the gas from the additional reservoir 18 via the emission line 6 (in the same way as explained above in the case of the liquid reservoir 4), and providing the liquid medium having a controlled concentration in a dissolved gas to the cell culture system 3 via the liquid inlet 16. This time, the liquid medium exiting the culture cell system 3 enters the liquid reservoir 4. When a major portion of the liquid medium has been transferred from the additional reservoir 18 to the liquid reservoir 4, the reservoirs may be switched again.

Alternatively, the recirculation step may comprise the transfer of the liquid medium exiting the cell culture system 3 from the liquid outlet 17, back to the liquid reservoir 4 for example by means of a recirculation pump as explained above.

The method according to the invention may be implemented to grow cells, including individual cells, organoids, cellular assemblies, organelles, organs-on-chips, and multicellular organisms. The cells may include, as an exemplary and non-exhaustive list, mammal cells, animal cells, plant cells, bacteria, protozoa, eukaryote cells, prokaryote cells, yeast cells, fungal cells, virus-containing cells, or any combination of such cells. Multicellular organisms may include animals, notably but not exclusively, laboratory model animals such as nematodes, embryos, notably non-human embryos such as fish embryos, flies, eggs, plants, genetically modified organisms (GMOs). The cell culture may also be performed in order to grow viruses.

The method according to the invention may also be implemented to perform biochemical reactions, or enzymatic reactions, or chemical reactions.

In addition, according to some preferred embodiments, the method according to the invention is used for a drug screening operation, or a toxicity screening operation.

EXAMPLES

The following examples illustrate the invention without limiting it.

Example 1 - experimental determination of pressure / flow rate

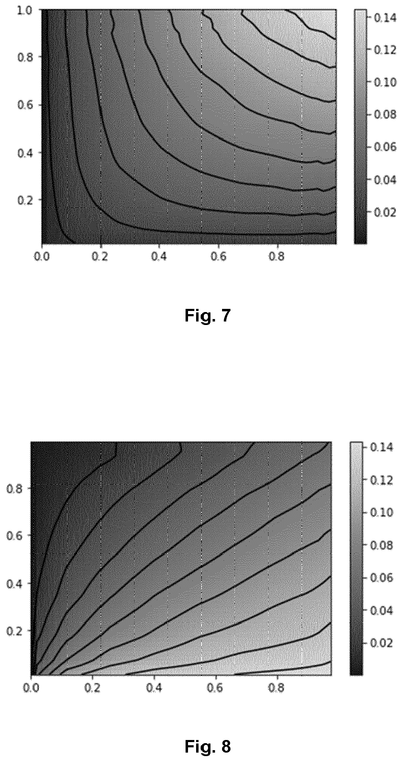

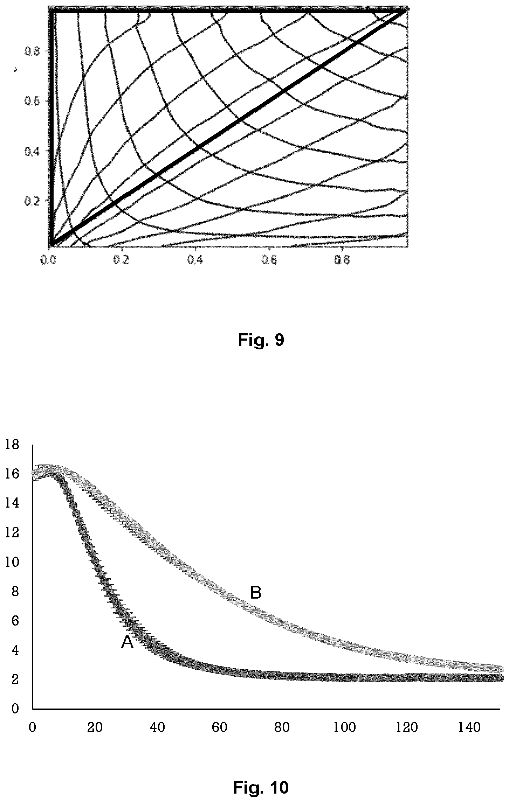

The setup of **figure 1** was used, using an oxygen container as a single gas source.
Gas flow rate measurements were performed using the gas flow sensor 13, using various admission valve and emission valve ratios, at a source gas pressure $P_{max}$=200 mbar above atmospheric pressure. As a result, experimental isoflow rate curves were obtained, as shown in **figure 7**.
Pressure measurements were performed using the pressure sensor 10' associated with the liquid reservoir 4, using various admission valve and emission valve ratios, at a source gas pressure $P_{max}$=200 mbar above atmospheric pressure. As a result, experimental isopressure curves were obtained, as shown in **figure 8.**
By superimposing both graphs, it can be seen that the isopressure and isoflow rate curves have intersections (**figure 9**).
In order to have a closer partial oxygen pressure to the one in atmospheric pressure, it may be desirable to maintain pressure below $P_{max}/2$. This corresponds to the upper part of the graph, identified with a triangle. In this section of the graph, the flowrate does not vary much as a function of the emission valve opening, which is graphically visible by the fact that isoflow rate curves are almost vertical. In other words, if the pressure to be controlled is sufficiently low compared to the input pressure (at most $P_{max}/2$), fixing an admission aperture will mostly determine the flow rate, while pressure can be changed by changing the emission valve aperture.
The experimental P/Q curves can be used as input for the control unit of the invention, so as to pre-open the valves according to the flow rate and pressure requested by the user before initiating a closed loop regulation in order to get rapidly close to the desired setpoint and achieve fast and efficient regulation.

Example 2 - effect of a variation of the gas flow rate on oxygen concentration

Still using the setup of **figure 1** equipped with a Presens flow-through oxygen sensor (FTCM-Pst7), oxygen was bubbled in the liquid reservoir 4 at two different gas flow rates, namely 0.133 L/min (A) and 0.3 L/min (B), and at the same relative pressure in the liquid reservoir 4 of 200 mbar ($Pout_{gas}$ being 1 bar). The result of the oxygen measurement is shown in **figure 10.**
It can be seen that the time required to reach a constant, equilibrium concentration of oxygen in the liquid medium (in this case approximately 2%) depends on the gas flow rate, a higher flow rate resulting in a shorter time to equilibrium.

Example 3 - cyclic variation of the concentration of dissolved gas

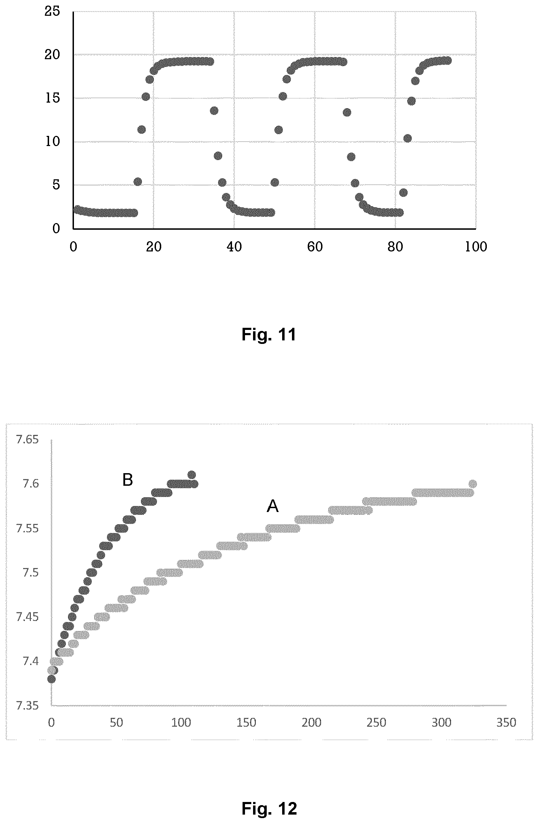

In this experiment, a cyclic modification of the oxygen content of the liquid medium was achieved. By switching between an air bottle and a gas bottle containing 2 % oxygen, cycles of gaseous oxygen were measured. This represents the possibility to switch between several mixed gas sources and alternately bubble in cell culture medium to apply cyclic concentration of dissolved oxygen.
The results are shown in **figure 11.** The oxygen concentration in the gaseous medium is made to oscillate between 2% and 20%.

Example 4 - effect of a variation of the gas flow rate on pH

A mixed-gas containing 5 % $CO_2$ and 20 % oxygen was bubbled in the liquid reservoir 4 at two different gas flow rates, namely 0.050 L/min (A) and 0.400 L/min (B), and at the same relative pressure in the liquid reservoir 4 of 500 mbar ($Pout_{gas}$ being 1 bar). The result of the pH measurement is shown in **figure 12.** It can be seen that the time required to reach a constant, equilibrium concentration of oxygen in the liquid medium (in this case approximately 2 %) depends on the gas flow rate, a higher flow rate resulting in a shorter time to equilibrium.

**Claims**

1. A device (2) for providing a liquid medium with a controlled flow rate and with a controlled concentration of a dissolved gas, the device (2) comprising:

- a liquid reservoir (4) provided with:

  - at least one gas inlet (7);
  - at least one gas outlet (8); and
  - at least one liquid outlet (9);

  - an admission line (5) configured to provide a gaseous medium into the liquid reservoir (4), the admission line (5) being connected to the at least one gas inlet (7) and comprising at least one control valve (12); and
  - an emission line (6) configured to withdraw the gaseous medium from the liquid reservoir (4), the emission line (6) being connected to the at least one gas outlet (8) and comprising at least one control valve (15).

2. The device (2) according to claim 1, wherein the emission line (6) is connected to a pressure controller (14).

3. The device (2) according to claim 1 or 2, wherein the liquid reservoir (4) is provided with at least one inlet (10) configured to receive a sensor (10'), preferably a pressure sensor (10').

4. The device (2) according to any one of claims 1 to 3, wherein the gaseous medium is air, oxygen, nitrogen or carbon dioxide or a mixture thereof.

5. The device (2) according to any one of claims 1 to 4, wherein the gas inlet (7) is configured for bubbling gaseous medium in the liquid medium contained in the liquid reservoir (4) and preferably comprises a tube having a first open extremity and a second open extremity, the second open extremity located in a lower part of the liquid reservoir (4) and/or wherein the liquid reservoir (4) comprises a liquid agitating element, such as a magnetic stirrer, and mechanical stirrer, an ultrasound or sound transducer, a vibrating element.

6. An assembly (1), comprising:

  - the device (2) according to any one of claims 1 to 5; and
  - a liquid utilization system (3) comprising at least one liquid inlet (16) and at least one liquid outlet (17), wherein the liquid outlet (9) of the device (2) is connected to the liquid inlet (16) of the liquid utilization system (3).

7. The assembly (1) according to claim 6, wherein the liquid utilization system (3) is a cell culture system (3) preferably selected from a microfluidic cell culture system, a millifluidic cell culture system or a nanofluidic cell culture system.

8. The assembly (1) according to any one of claims 6 or 7, comprising an additional reservoir (18) connected to a pressure controller (14') and comprising a liquid inlet (21) connected to the liquid outlet (17) of the liquid utilization system (3) and/or the assembly comprising a flow restrictor configured for modifying the hydraulic resistance for the liquid medium downstream of the liquid reservoir (4).

9. The assembly (1) according to claim 8, further comprising a recirculation module (24) configured to connect the additional reservoir (18) to the liquid inlet (16) of the liquid utilization system (3), to the admission line (5) and to the emission line (6).

10. A method for providing a liquid medium with a controlled flow rate and with a controlled concentration in a dissolved gas, implemented in the assembly (1) according to any one of claims 6 to 9, the method comprising the steps of:

  - providing the liquid reservoir (4) with a liquid medium;
  - flowing gaseous medium into the liquid reservoir (4) via the admission line (5) and out of the liquid reservoir (4) via the emission line (6), so as to apply a pressure in the liquid reservoir (4);
  - flowing the liquid medium from the liquid reservoir (4) to the liquid utilization system (3) due to the pressure in the liquid reservoir (4).

11. The method according to claim 10 comprising a step of applying a counter-pressure at the liquid outlet (17) of the liquid utilization system (3) and/or comprising a step of modifying the hydraulic resistance downstream of the liquid reservoir (4).

12. The method according to any one of claims 10 or 11, comprising a step of varying the flow rate of the liquid medium while maintaining the concentration of the dissolved gas in the liquid medium substantially constant.

13. The method according to any one of claims 10 to 12, comprising a step of controlling the control valve(s) (12) on the admission line (5) and/or the control valve (15) on the emission line (6) to adjust at least one concentration of dissolved gas, preferably oxygen and/or carbon dioxide, in the liquid medium to a predetermined value, preferably using a closed loop regulation and/or the method comprising a step of controlling the control valve(s) (12) on the admission line (5) and/or the control valve (15) on the emission line (6) to adjust the pH of the liquid medium to a predetermined value, preferably using a closed loop regulation and/or the method comprising a step of controlling a counter-pressure at the liquid outlet (17) of the liquid utilization system (3) to adjust the flow rate of the liquid medium from the liquid reservoir (4) to the liquid utilization system (3), preferably using a closed loop regulation or the method comprising controlling a hydraulic resistance downstream of the liquid reservoir (4) to adjust the flow rate of the liquid medium from the liquid reservoir (4) to the liquid utilization system (3), preferably using a closed loop regulation.

14. A method of culturing cells in a cell culture system (3), wherein the liquid utilization system (3) is provided with a liquid medium according to the method of one of claims 10 to 13.

15. A non-transitory computer readable storage medium having stored thereon instructions that, when executed, cause at least one computing device to control the device (2) of one of claims 1 to 5 or the assembly (1) of one of claims 6 to 9 so as to carry out the method of any one of claims 10 to 14.

**Amended claims in accordance with Rule 137(2) EPC.**

1. A device (2) for providing a liquid medium with a controlled flow rate and with a controlled concentration of a dissolved gas to a liquid utilization system (3) the device (2), comprising:

   - a liquid reservoir (4) provided with:

       - at least one gas inlet (7);
       - at least one gas outlet (8); and
       - at least one liquid outlet (9);

   - an admission line (5) configured to provide a gaseous medium into the liquid reservoir (4), the admission line (5) being connected to the at least one gas inlet (7) and comprising at least one control valve (12); and
   - an emission line (6) configured to withdraw the gaseous medium from the liquid reservoir (4), the emission line (6) being connected to the at least one gas outlet (8) and comprising at least one control valve (15);

   wherein the device (2) is configured for:

   - flowing gaseous medium into the liquid reservoir (4) via the admission line (5) and out of the liquid reservoir (4) via the emission line (6), so as to apply a pressure in the liquid reservoir (4); and
   - flowing the liquid medium from the liquid reservoir (4) to the liquid utilization system (3) due to the pressure in the liquid reservoir (4).

2. The device (2) according to claim 1, wherein the emission line (6) is connected to a pressure controller (14).

3. The device (2) according to claim 1 or 2, wherein the liquid reservoir (4) is provided with at least one inlet (10) configured to receive a sensor (10'), preferably a pressure sensor (10').

4. The device (2) according to any one of claims 1 to 3, wherein the gaseous medium is air, oxygen, nitrogen or carbon dioxide or a mixture thereof.

5. The device (2) according to any one of claims 1 to 4, wherein the gas inlet (7) is configured for bubbling gaseous medium in the liquid medium contained in the liquid reservoir (4) and preferably comprises a tube having a first open extremity and a second open extremity, the second open extremity located in a lower part of the liquid reservoir (4) and/or wherein the liquid reservoir (4) comprises a liquid agitating element, such as a magnetic stirrer, and mechanical stirrer, an ultrasound or sound transducer, a vibrating element.

6. An assembly (1), comprising:

   - the device (2) according to any one of claims 1 to 5; and
   - a liquid utilization system (3) comprising at least one liquid inlet (16) and at least one liquid outlet (17),

   wherein the liquid outlet (9) of the device (2) is connected to the liquid inlet (16) of the liquid utilization system (3).

7. The assembly (1) according to claim 6, wherein the liquid utilization system (3) is a cell culture system (3) preferably selected from a microfluidic cell culture system, a millifluidic cell culture system or a nanofluidic cell culture system.

8. The assembly (1) according to any one of claims 6 or 7, comprising an additional reservoir (18) connected to a pressure controller (14') and comprising a liquid inlet (21) connected to the liquid outlet (17) of the liquid utilization system (3) and/or the assembly comprising a flow restrictor configured for modifying the hydraulic resistance for the liquid medium downstream of the liquid reservoir (4).

9. The assembly (1) according to claim 8, further comprising a recirculation module (24) configured to connect the additional reservoir (18) to the liquid inlet (16) of the liquid utilization system (3), to the admission line (5) and to the emission line (6).

10. A method for providing a liquid medium with a controlled flow rate and with a controlled concentration in a dissolved gas, implemented in the assembly (1) according to any one of claims 6 to 9, the method comprising the steps of:

    - providing the liquid reservoir (4) with a liquid medium;
    - flowing gaseous medium into the liquid reservoir (4) via the admission line (5) and out of the liquid reservoir (4) via the emission line (6), so as to apply a pressure in the liquid reservoir (4);
    - flowing the liquid medium from the liquid reservoir (4) to the liquid utilization system (3) due to the pressure in the liquid reservoir (4).

11. The method according to claim 10 comprising a step of applying a counter-pressure at the liquid outlet (17) of the liquid utilization system (3) and/or comprising a step of modifying the hydraulic resistance downstream of the liquid reservoir (4).

12. The method according to any one of claims 10 or 11, comprising a step of varying the flow rate of the liquid medium while maintaining the concentration of the dissolved gas in the liquid medium substantially constant.

13. The method according to any one of claims 10 to 12, comprising a step of controlling the control valve(s) (12) on the admission line (5) and/or the control valve (15) on the emission line (6) to adjust at least one concentration of dissolved gas, preferably oxygen and/or carbon dioxide, in the liquid medium to a predetermined value, preferably using a closed loop regulation and/or the method comprising a step of controlling the control valve(s) (12) on the admission line (5) and/or the control valve (15) on the emission line (6) to adjust the pH of the liquid medium to a predetermined value, preferably using a closed loop regulation and/or the method comprising a step of controlling a counter-pressure at the liquid outlet (17) of the liquid utilization system (3) to adjust the flow rate of the liquid medium from the liquid reservoir (4) to the liquid utilization system (3), preferably using a closed loop regulation or the method comprising controlling a hydraulic resistance downstream of the liquid reservoir (4) to adjust the flow rate of the liquid medium from the liquid reservoir (4) to the liquid utilization system (3), preferably using a closed loop regulation.

14. A method of culturing cells in a cell culture system (3), wherein the liquid utilization system (3) is provided with a liquid medium according to the method of one of claims 10 to 13.

15. A non-transitory computer readable storage medium having stored thereon instructions that, when executed, cause at least one computing device to control the device (2) of one of claims 1 to 5 or the assembly (1) of one of claims 6 to 9 so as to carry out the method of any one of claims 10 to 14.

**Fig. 1**

**Fig. 2**

**Fig. 3**

**Fig. 4**

**Fig. 5**

Fig. 6A

Fig. 6B

Fig. 7

Fig. 8

**Fig. 9**

**Fig. 10**

**Fig. 11**

**Fig. 12**

Europäisches
Patentamt

European
Patent Office

Office européen
des brevets

# EUROPEAN SEARCH REPORT

Application Number

EP 20 30 6145

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2017/198246 A1 (NIAZI SARFARAZ K [US]) 13 July 2017 (2017-07-13) * paragraph [0027]; figure 1 * | 1-15 | INV. C12M3/06 B01F3/04 C12M1/00 C12M1/34 |
| A | US 2007/217964 A1 (JOHNSON TIMOTHY J [US] ET AL) 20 September 2007 (2007-09-20) * paragraph [0011] - paragraph [0014] * * paragraph [0044] - paragraph [0059] * | 7-9 | |
| A | JP 2008 271862 A (IHI CORP) 13 November 2008 (2008-11-13) * paragraph [0020] - paragraph [0029] * * figure 1 * | 1-15 | |
| A | JP 2005 091352 A (NAT INST FOR MATERIALS SCIENCE) 7 April 2005 (2005-04-07) * paragraphs [0019], [0020], [0024]; figure 1 * | 1-6 | |
| A | US 2008/299539 A1 (LEE HARRY [US] ET AL) 4 December 2008 (2008-12-04) * paragraph [0010] - paragraph [0012] * * paragraph [0074] - paragraph [0075] * * figure 13 * | 7-9 | |

| | | |
|---|---|---|
| | | TECHNICAL FIELDS SEARCHED (IPC) |
| | | C12M B01F |

The present search report has been drawn up for all claims

| Place of search | Date of completion of the search | Examiner |
|---|---|---|
| Berlin | 16 March 2021 | Cubas Alcaraz, Jose |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.................................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 20 30 6145

This annex lists the patent family members relating to the patent documents cited in the above-mentioned European search report.
The members are as contained in the European Patent Office EDP file on
The European Patent Office is in no way liable for these particulars which are merely given for the purpose of information.

16-03-2021

| Patent document cited in search report | | | Publication date | Patent family member(s) | | | Publication date |
|---|---|---|---|---|---|---|---|
| US 2017198246 | A1 | | 13-07-2017 | NONE | | | |
| US 2007217964 | A1 | | 20-09-2007 | CA | 2637613 | A1 | 30-08-2007 |
| | | | | EP | 1991655 | A2 | 19-11-2008 |
| | | | | JP | 2009527225 | A | 30-07-2009 |
| | | | | US | 2007217964 | A1 | 20-09-2007 |
| | | | | WO | 2007098027 | A2 | 30-08-2007 |
| JP 2008271862 | A | | 13-11-2008 | JP | 5125210 | B2 | 23-01-2013 |
| | | | | JP | 2008271862 | A | 13-11-2008 |
| JP 2005091352 | A | | 07-04-2005 | JP | 4635247 | B2 | 23-02-2011 |
| | | | | JP | 2005091352 | A | 07-04-2005 |
| US 2008299539 | A1 | | 04-12-2008 | US | 2008299539 | A1 | 04-12-2008 |
| | | | | WO | 2007044699 | A1 | 19-04-2007 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20160312166 A
- CN 108148749
- KR 101294521
- WO 2019191685 A
- US 20130295551 A
- WO 2014082377 A
- RU 2587628 C1